# EUROPEAN PATENT SPECIFICATION

(11) **EP 1 384 786 B1**
(45) Date of publication and mention of the grant of the patent: **27.01.2010**
(21) Application number: 02708770.9
(22) Date of filing: 02.04.2002
(51) Int. Cl.: C12P 19/00, C07H 1/00, C12N 15/00

(54) **PROCESS FOR PRODUCING OLIGOSACCHARIDE CHAINS**
VERFAHREN ZUR HERSTELLUNG VON OLIGOSACCHARIDKETTEN
METHODE DE PRODUCTION DE CHAINES D'OLIGOSACCHARIDES

(30) Priority: 02.04.2001 JP 2001103737
(43) Date of publication of application: 28.01.2004
(73) Proprietor: GLYCOMEDICS, INC., Minato-ku Tokyo 105-0001 (JP)
(72) Inventor: SATO, Toshinori, Yokohama-shi, Kanagawa 223-0052 (JP); SANO, Emiko, Yokohama-shi, Kanagawa 241-0825 (JP)
(74) Representative: Coleiro, Raymond
(86) International application number: PCT/JP2002/003310
(87) International publication number: WO 2002/081723

(56) References cited:
- EP-A- 0 565 241
- EP-A2- 0 764 719
- WO-A-00/29603
- FR-A- 2 796 082
- JP-A- 2000 247 992
- US-A- 5 294 546
- SATO TOMONORI: 'Saibo o riyo shita tosa kobunshi gosei tosa polymer no sekkei' POLYMER PREPRINTS vol. 49, no. 13, 2000, JAPAN, pages 4030 - 4031, XP002951033
- YAMAGATA T. ET AL.: 'Azido glycoside primer: a versatile building block for the biocombinatorial synthesis of glycosphingolipid analogues' CARBOHYDR. RES. vol. 329, no. 4, 01 December 2000, pages 755 - 763, XP004228032
- YAMAGATA T. ET AL.: 'Glycosylation of amphipathic lactoside primers with consequent inhibition of endogenous glycosphingolipid synthesis' J. BIOCHEM. vol. 124, no. 1, July 1998, pages 148 - 156, XP002951035
- SATO SEIJI: 'Dobutsu no soshiki baiyo 2 dobutsu saibo no tairyo baiyo to sono riyo' THE HERIDITY vol. 38, no. 8, 1984, pages 72 - 79, XP002951036

## Description

### Technical Field

The present invention relates to a method for producing oligosaccharides for use in pharmaceutical drugs, medicals, glyco chips and the like.

### Background Art

Cells in the living organism are deeply involved in cellular functions by expressing specific sugar chains at every process of fertilization, development, differentiation, propagation, cell death and the like. In addition, sugar chains are receptors for many toxins, viruses, bacteria etc. and are also attracting attention as tumor markers. Recently, their associations with amyloid proteins, that have been implicated in the etiology of Alzheimer's disease and with metastasis of cancer cells have also been reported.

Animal cells express different types of sugar chains peculiar to each to them, and their functions have been elucidated in recent years. For example, B16 melanoma cells express sugar chains of the GM3 type (Komori, H. et al., FEBS Letters 374: 299-302, 1995), PC12 cells express sugar chains of the Gb3 type (Shimamura, M. et al., J. Biol. Chem., 263.24: 12124-12128, 1998), and COS-7 cells express sugar chains of the A series ganglioside (Hisae Anyouji et al., Proceedings of Japan Chemical Society in 2000). Gb3 sugar chains provide receptor for Vero toxin, and an oligosaccharide having sialyl galactose and the GM1 type oligosaccharide are known to be a receptor for influenza virus and cholera toxin, respectively. Furthermore, as the GD1a type oligosaccharide are involved in cell adhesion, their association with cancer metastasis is also under discussion. Thus, sugar chains have a variety of functions and the functional analyses of sugar chains are indispensable for future medicine, medical use and diagnosis of diseases, and thus there is a great demand for the construction of saccharide library comprising a variety of sugar chains. Up to now, available saccharide library have not yet to be constructed as the synthetic technology of sugar chains is not established fully.

On the other hand, although yeast has been used as a host for efficient production of useful substances, it also has a biosynthetic system for sugar chains, and various sugar chains have been synthesized therewith. However, glycoproteins produced by yeast have bound thereto sugar chains termed as the high-mannose type, which exhibits antigenicity in the humans making them unsuitable as pharmaceutical drugs.

Also, plants including various fungi contain β-glucan, a kind of dietary fiber called polysaccharide. This β-glucan is known to have an immunoenhancing effect.

Synthetic methods of sugar chains include chemical synthesis and those using enzymes. Although chemical synthesis requires many reaction steps and special technology and an enormous time and personnel cost in order to obtain one natural oligosaccharide, its yield is low nevertheless, and thus oligosaccharides obtained by chemical synthesis have a drawback of being costly as compared to extraction from a natural origin.

Also, the preparation of oligosaccharides by enzymatic methods involves a method using hydrolytic enzymes and one using glycosyltransferases. However, enzymes that can be used are limited, and therefore it is practically impossible, at present, to freely produce the desired oligosaccharides. Furthermore, since raw materials for reaction are limited, very expensive and the like, the preparation of saccharide library has not reached a practical stage.

In sugar chain synthesis using animal cells, it is known that sugar chain extension takes place when a saccharide primer of a simple structure is administered to cultured cells, the oligosaccharides produced are released into the culture medium, and, by changing the type of cultured cells, oligosaccharides of different structures may be synthesized (Nakajima H. et al., J. Biochem. 124: 148-156, 1998). In this method, by administering only saccharide primers that can be synthesized at large quantities and with ease to cultured cells, oligosaccharide functioning in the living organism can only be produced, and thus the method has an advantage that very high quality pools of saccharide library can be obtained. At present, however, these experiments for producing sugar chains are being carried out in monolayer cultures using culture dishes, and thus it is difficult to provide quantities required to construct saccharide library.

So far the production of useful physiologically active substances by using animal cells was difficult, especially scale up of culturing of anchorage dependent animal cells was difficult. Recently, although an attempt of culturing COS-7 cells in a small scale microcarrier culture and producing oligosaccharides by administering saccharide primer was reported, scale-up culturing while maintaining cell functions of cells other than anchorage dependent cancer cells such as COS-7 cells was very difficult, and the microcarrier culture method could not be applied to all cells.

JP2000-247992-A discloses a new class of saccharide chain primers for saccharide chain biosynthesis, and refers to the use of B16 mouse melanoma cells, rat PC12 cells, mouse Neuro2a cells, rat RBL-2H3 cells, human MOLT-4 cells, and human HL-60 cells.

Hisae et al. (2001) Journal of Molecular Catalysis B Enzymatic 14:66 discloses that, for constructing a saccharide library, production of oligosaccharides was carried out by adding simple saccharide primers (n-dodecyl lactoside) to living cells such as COS-7.

### Disclosure of the Invention

The present invention intends to provide a method of efficiently producing, from animal cells etc., useful saccharide library that are likely to be used in pharmaceutical drugs, medicals, glyco chips and the like.

The conventional monolayer cultures can provide a small amount of oligosaccharide produced in the cells for analysis of the structure but they cannot provide oligosaccharides as a raw material on a commercial basis and cannot provide, in large quantities, a variety of oligosaccharides for the construction of saccharide library. For that purpose, a simple high-density, large-scale culture method is required. If an experimental system in which saccharide primers are administered using an industrially feasible high-density large-scale culture can be actually performed, useful oligosaccharide could be produced in large quantities, and saccharide library could be constructed. Moreover, as a variety of oligosaccharides can simultaneously be produced at one time using tumor cells from various animals grown in a large-scale high-density culture, an energy-saving and efficient production of sugar chains can be accomplished. Recently, from a viewpoint of material production having a light burden on environment as well, though organic solvents are used at some of the processes for the synthesis of saccharide primers and the extraction of products, organic solvents are not used in other steps, and, thus, material production having a light burden on environment can be attained, compared to other methods.

A large-scale culture that has become an important issue in material production using animal cells can be attained to some degree for hybridoma cells that produce specific antibodies and other suspension cell lines using tank culture that permits scale-up, but the scale-up of anchorage dependent cells such as human diploid fibroblast cells and other adherent cell lines is difficult for the difficulty of the equipment for a large-scale culture, and thus there is a great demand for the establishment of batch culture technology of adherent cells that maintain cellular functions.

The above objective can be attained by the present invention. The present invention is a method as defined in the claims for producing oligosaccharides comprising giving saccharide primers to human cells cultured using a high-density large-scale culture method.

Preferred human cells include in particular diploid fibroblasts or vascular endothelial cells.

There can also be used cells containing a vector that has integrated DNA encoding a sugar chain biosynthetic enzyme. As sugar chain biosynthetic enzymes, human-type enzymes are preferred.

The above high-density culture method is preferably a microcarrier culture method, a culture apparatus using a cell immobilization disk, a culture system using a hollow fiber module, or a spinner culture for suspension cells.

### Brief Explanation of the Drawings

Figure 1 is a drawing showing a migration pattern of high performance thin layer chromatography (HPTLC) of oligosaccharides produced in Example 1.
Figure 2 is a chart showing the analytical result by MALDI-TOF MS of oligosaccharides X1, X2, and X4, as well as the substrate saccharide primer shown in Figure 1 and Table 1.
Figure 3 is a chart showing the result by MALDI-TOF MS of oligosaccharides X3 and X5, as well as the substrate saccharide primer shown in Figure 1 and Table 1.
Figure 4 is showing a growing curve of human vascular endothelial cells in a microcarrier culture until the addition of the substrate saccharide primer.
Figure 5 shows a migration pattern of high performance thin layer chromatography (HPTLC) of oligosaccharides produced in Example 3. Lane 1 represents the substrate saccharide primer, lanes 2-4 and lanes 5-7 represent the result when Cytodex-1 and Cytodex-3 were used as microcarriers respectively, and in these lanes, lanes 2 and 5 represent the result after culturing for 24 hours, lanes 3 and 6 for 48 hours, and lanes 4 and 7 for 72 hours. Lane 8 is the result when cultured in a dish culture for 48 hours.
Figure 6 is a chart showing the result of structural analysis by MALDI-TOF MS of oligosaccharides X1, X2, and X3 shown in Figure 5 and Table 3.
Figure 7 is a graph showing the relative amounts of oligosaccharides X1 (Gb3-C12) and X2 (Gal-Gb3-C12) produced in a dish culture using a culture dish having a diameter of 100 mm and a culture using Cytodex-1 and Cytodex-3.

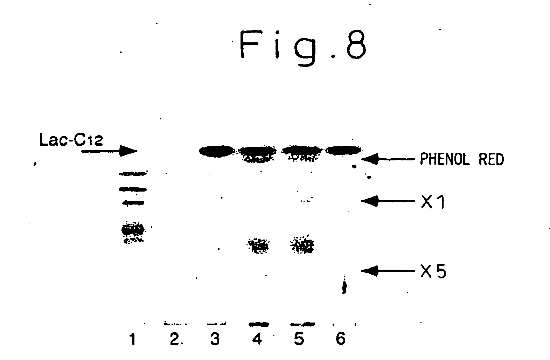

Figure 8 shows the result of high performance thin layer chromatography of concentrates of the supernatant obtained from a microcarrier culture using Cytodex-1 (lane 4) or Cytodex-3 (lane 5) and the supernatant obtain from a monolayer culture in Example 4. Lane 1 represents the ganglioside standard and lane 6 represents the substrate saccharide primer.
Figure 9 is a chart showing the result by MALDI-TO MS of oligosaccharides shown in Table 4 produced in Example 4.
Figure 10 is a graph showing the relative amounts of the oligosaccharides X1 (GM3 type) produced in a dish culture using a culture dish having a diameter of 100 mm and cultures using Cytodex-1 and Cytodex-3. Best Mode for Carrying Out the Invention

Cells for use in the production of oligosaccharide of the present invention may contain vectors that have integrated thereinto DNA encoding a sugar chain synthase.

High-density culture methods of cells for use in the present invention include microcarrier culture methods, culture apparatus using cell-immobilization disks, culture systems using hollow-fiber modules, spinner cultures of suspension cells, multitray culture systems, methods using roller bottles, methods of immobilizing cells in microcapsules and the like, and preferably microcarrier culture methods, culture systems using cell-immobilization disks, culture systems using hollow-fiber modules, or spinner cultures of suspension cells are used.

Microcarriers that are preferably used include those in which matrix material comprises collagen, gelatin, cellulose, cross-linked dextran or resins such as polystyrene, and charged groups are dimethylaminopropyl, dimethylaminoethyl, trimethylhydroxyaminopropyl, or negatively charged groups. There can also be used matrix materials coated with collagen or gelatin. Commercial products include "Cytodex-1, Amersham Pharmacia Biotech AB" and "Cytodex-3, Amersham Pharmacia Biotech AB" in which dimethylaminoethyl has been added to cross-linked dextrans. As hollow fibers, there are those that use modified cellulose ("Vitafiber", Amicon Inc.).

For microcapsules, there is known a method in which collagen or sodium alginate that form water permeable gels are used and cells are embedded in therein (A. Klausner, Bio/Technol., 1: 736, 1983).

A small-scale culture for microcarriers is started by placing PBS(-) containing microcarriers into a spinner flask, which is steam sterilized and the PBS(-) is replaced with a culture medium, and then inoculating cells therein. The culture medium is replaced at an appropriate interval, and when the cells are propagated to confluence on the microcarriers, a saccharide primer is administered. For human vascular endothelial cells etc. that require growth factors for propagation and survival, vascular endothelial growth factor (VEGF) or fibroblast growth factor (FGF) etc. are added to the culture medium, but it is known that a large-scale culture of endothelial cells is difficult.

In the microcarrier culture, one culture bottle of a 200 ml scale can provide the number of cells equivalent to that cultured in 100 Petri dishes with an internal diameter of 100 mm and, besides, the cell number per unit volume of liquid is about four times more dense. Therefore, it has an advantage that the dosage of saccharide primers can be small and novel oligosaccharides can be detected that cannot be detected in the cells in Petri dishes.

Saccharide primers for use in the present invention include, but are not limited to, analogs in which hydrophobic chains have been attached to lactose or galactose that were made in order to simulate the structure of lactosylceramide, a synthetic precursor in vivo for glycolipid sugar chains, or saccharide primers having N-acetylglucosamine, N-acetylgalactosamine or the like. A method of preparing saccharide primers is described in Japanese Unexamined Patent Publication (Kokai) No. 2000-247992.

In order to generate oligosaccharides from cultured cells, 10-100 µM of a saccharide primer is administered using a serum-free medium or a low-concentration serum medium to confluent cells, which are cultured at 37°C for 1-5 days to obtain a production stock containing extended sugar chains. The culture supernatant is harvested, and subjected to concentration, separation, and structural analysis to obtain a variety of oligosaccharide library. Depending on the type of cells, the type and the dosage of saccharide primers, the culture medium and the culture days are different and, thus, to find an optimum culture condition for each cell leads to more efficient production of oligosaccharides.

Oligosaccharides contained in the harvested liquid are concentrated and separated using affinity chromatography, ultra filtration, ammonium sulfate precipitation, or the like, and the structure is analyzed using high performance thin layer chromatography (HPTLC) and MALDI-TOF MS. After blotting to high performance thin layer chromatography, unknown substances are subjected to enzyme treatment, and the product obtained is subjected to analysis of composition in order to estimate the structure.

### Examples

### Example 1: Production of oligosaccharides using human normal fibroblasts

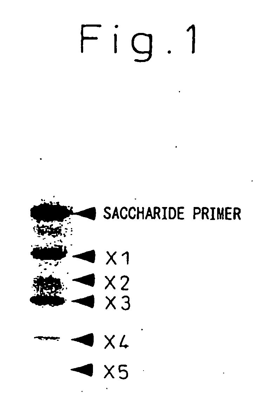

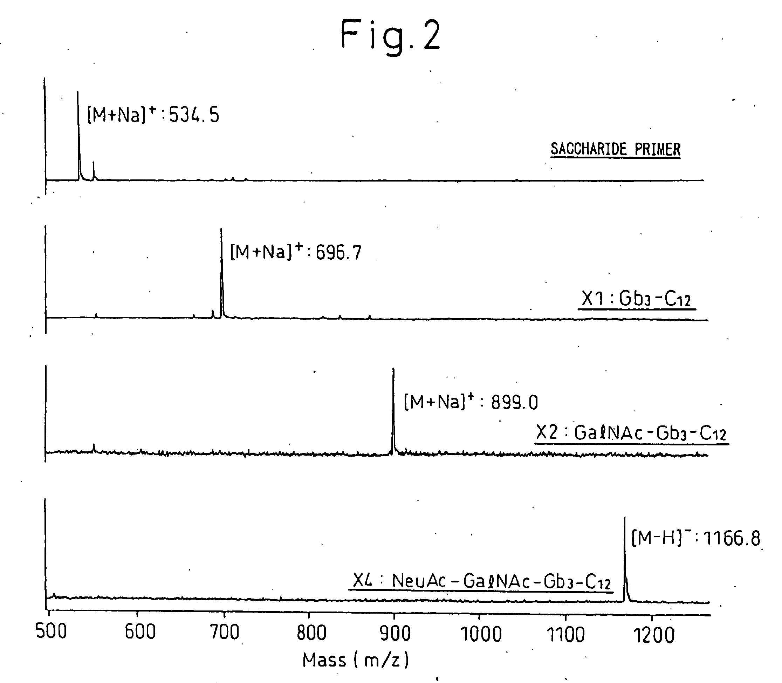

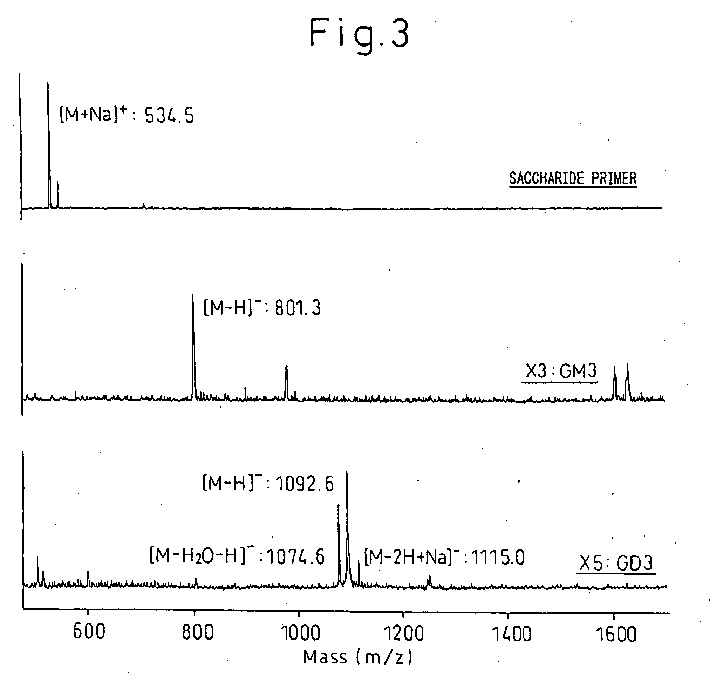

In an experiment for synthesizing sugar chains by dish culture, human normal fibroblast cells were propagated using Eagle's minimum essential medium (MEM) containing 10% fetal calf serum (FCS) in a 75 cm² culture flask. For the microcarrier culture, 4 x 10⁷ cells that were propagated by dish culture were inoculated into a 500 ml spinner flask containing 200 ml Cytodex-1 (Amersham Pharmacia Biotech AG) prepared at 0.3 w/v%, and cultured by stirring at 100-150 rpm. Cell counts when they reached confluence in the 75 cm² flask and in the microcarrier culture were 5 x 10° cells/flask and 4 x 10⁸ cells/bottle, respectively. The culture medium was replaced with a phenol red-free and serum-free Eagle's MEM, and the cells were treated with a 50 µM saccharide primer Gal-Glc-C12. Culturing was further continued. Four days later, the culture supernatant was harvested, and concentrated by high performance liquid chromatography (HPLC) using a C18 column, and then the structure of sugar chains was analyzed by HPTLC and MALDI-TOF MS. Figure 1 shows a migration pattern (band) of HPTLC, and Figure 2 and Figure 3 show the analytical result of each band by MALDI-TOF MS. The structures of oligosaccharide obtained from the lactoside primer (Gal-Glc-C12) using a microcarrier culture are shown in Table 1. In the dish culture, the oligosaccharides of (X1) and (X3) were only confirmed.

**Table 1. Oligosaccharides produced by human fibroblasts**

| (microcarrier culture) | | |
|---|---|---|
| (X1) | Gal-Gal-Glc-C12 | (Gb3 type) |
| (X2) | GalNAc-Gal-Gal-Glc-C12 | (Gb4 type) |
| (X4) | NeuNAc-GalNAc-Gal-Gal-Glc-C12 | (NeuNAc-Gb4 type) |
| (X3) | NeuNAc-Gal-Glc-C12 | (GM3 type) |
| (X5) | NeuNAc-NeuNAc-Gal-Glc-C12 | (GD3 type) |

### Example 2: Production of oligosaccharides using human vascular endothelial cells

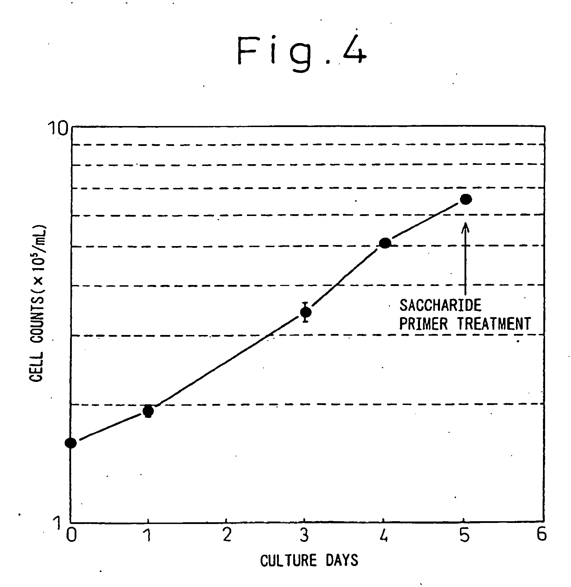

Endothelial cells isolated from a human umbilical cord vein were cultured in a collagen-coated flask (25 cm², 75 cm²), and were used in an experiment for the synthesis of oligosaccharides in dish culture. The culture medium was M199 medium to which 10% fetal calf serum (FCS) and, as a growth factor, 10 ng/ml basic fibroblast growth factor (basic FGF) were used. In the microcarrier culture of endothelial cells, cells propagated in a collagen-coated flask were inoculated at about 1.5 x 10⁵ cells/ml to a spinner flask (200 ml) in which 0.6 g of gelatin-coated microcarriers had been placed and sterilized, and cultured at a stirring speed of 200 rpm using the same medium as the dish culture. The growth curve of human endothelial cells in the microcarrier culture is shown in Figure 4. After the cells had grown to confluence, the medium was replaced with a phenol red-free M199 medium (1% FCS), and the saccharide primer Gal-Glc-C₁₂ was treated. 48 hours later, the culture supernatant was harvested. The culture supernatant was concentrated by reverse phase HPLC using a C18 column, and subjected to structural analysis using HPTLC and MALDI-TOF MS. The result is shown in Table 2. In the dish culture, oligosaccharide of (1) and (4) were only confirmed.

**Table 2: Oligosaccharides produced by human vascular endothelial cells**

| | | |
|---|---|---|
| (1) | Gal-Gal-Glc-C12 | (Gb3 type) |
| (2) | GalNAc-Gal-Gal-Glc-C12 | (Gb4 type) |
| (3) | NeuNAc-GalNAc-Gal-Gal-Glc-C12 | (NeuNAc-Gb4 type) |
| (4) | NeuNAc-Gal-Glc-C12 | (GM3 type) |

### Reference Example 3: Production of oligosaccharides using rat PC12

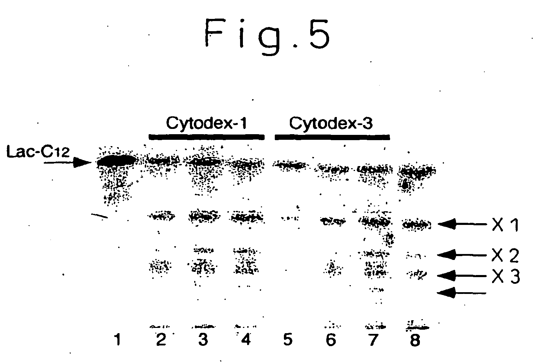

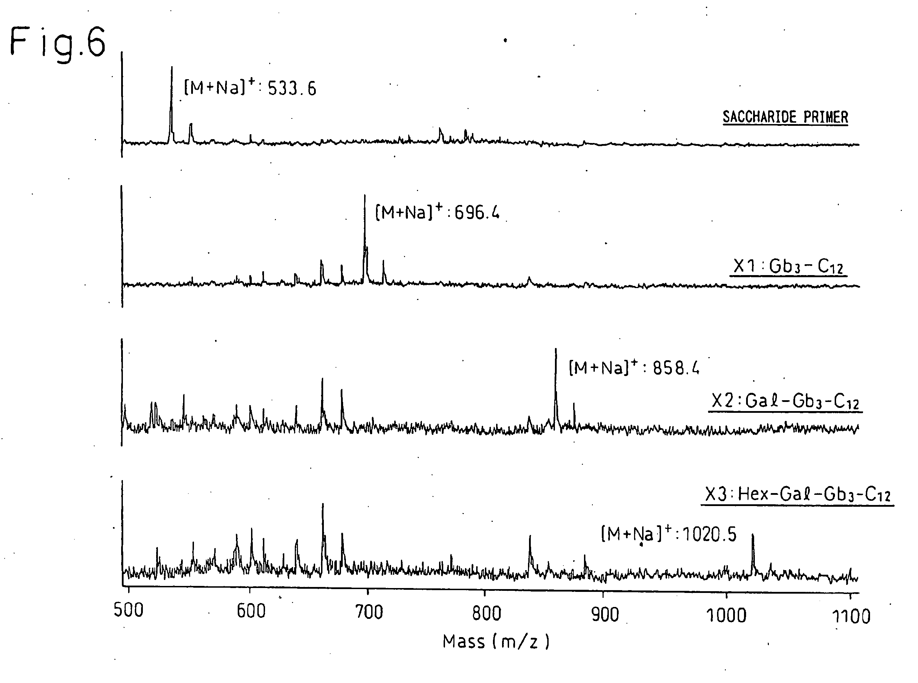

Rat PC12 cells were cultured in a 75 cm² flask and a microcarrier culture method using Cytodex-1 and Cytodex-3, and a saccharide primer Gal-Glc-C12 was administered to synthesize sugar chains. Figure 5 shows a migration pattern of high performance thin layer chromatography of the fractions of the culture medium. The result of structural analysis of these bands by MALDI-TOF MS is shown in Figure 6. The structure of sugar chains obtained in the analysis of the fractions obtained by the microcarrier culture is shown in Table 3.

**Table 3. Oligosaccharides produced by rat PC12 cells**

| | | |
|---|---|---|
| (X1) | Gal-Gal-Glc-C12 | (Gb3 type) |
| (X2) | Gal-Gal-Gal-Glc-C12 | (Ga1-Gb3 type) |
| (X3) | Hex-Gal-Gal-Gal-Glc-C12 | (Hex-Gal-Gb3 type) |

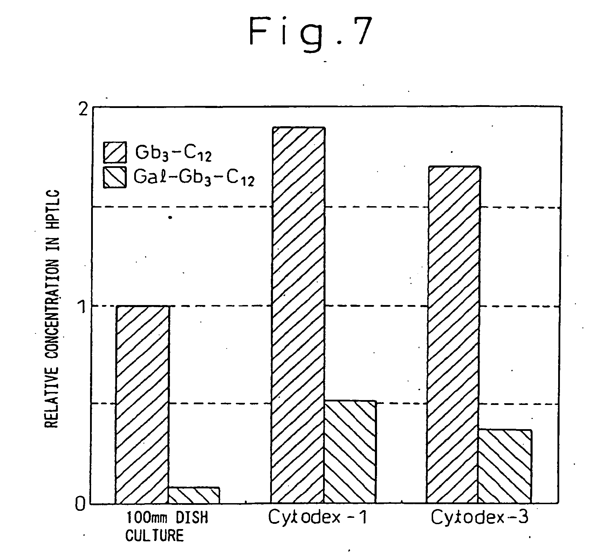

The amounts produced of two types of oligosaccharide synthesized in the dish culture and the microcarrier culture were quantitated by analyzing the thin layer chromatography stained with resorcinol/HCl by a densitometer, and the result is shown in Figure 7. It was shown that in the microcarrier culture the amount synthesized of oligosaccharides is increased by 2-5-fold per unit amount of liquid as compared to the dish culture.

### Reference Example 4: Production of oligosaccharides using COS-7

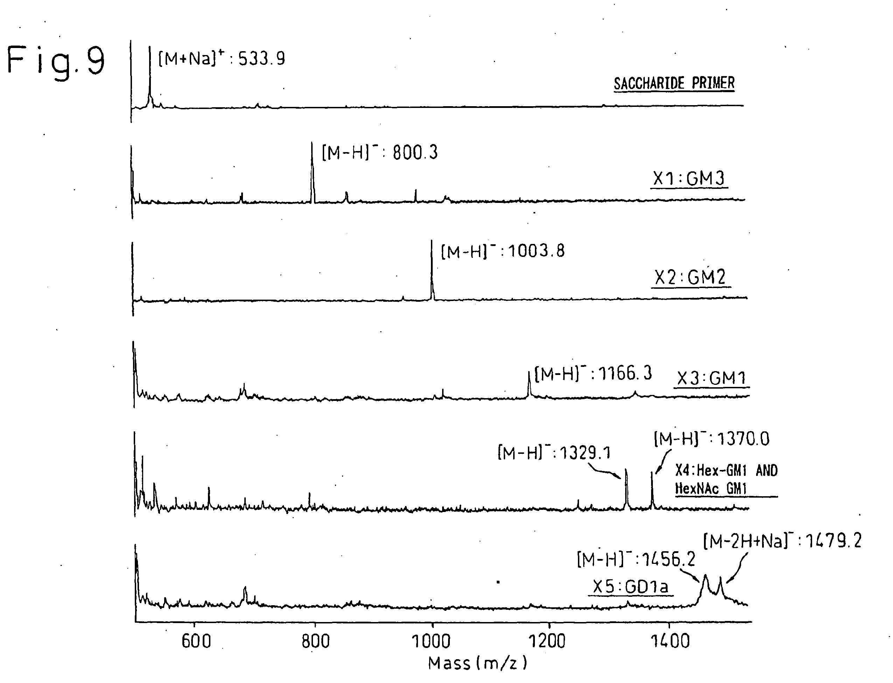

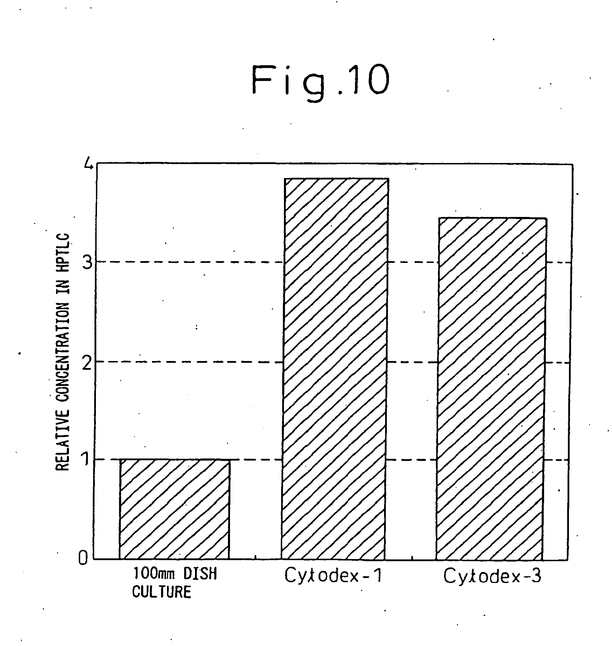

COS-7 cells were cultured in a Petri dish having a diameter of 100 mm and in the microcarrier culture using Cytodex-1 and Cytodex-3, and a saccharide primer Gal-Glc-C12 was administered to synthesize sugar chains. The cells were cultured in a culture medium in which 10% fetal calf serum (FCS) was added to Dulbecco's MEM. For the microcarrier culture, 0.6 g each of Cytodex-1 and Cytodex-3 was placed in a spinner culture flask and then sterilized, to which 200 ml of the culture medium was placed, and cells that had been propagated by dish culture were inoculated thereinto at a concentration of about 2 x 10⁵ cells/ml. After the cells have grown to confluence, the culture medium was replaced with a serum-free and phenol red-free medium. 50 µM of a saccharide primer Gal-Glc-C12 was administered, and culturing was continued. The culture supernatant was harvested, and concentrated by reverse phase HPLC using a C18 column, and then the oligosaccharides produced were analyzed by HPTLC and MALDI-TOF MS. Table 4 shows the analytical result of oligosaccharides obtained by the microcarrier culture using Cytodex-1. Figure 9 shows the result of analysis by MALDI-TOF MS. Figure 10 shows a comparison of the amount produced of a sugar chain of GM3 type by dish culture (Petri dish) and the microcarrier culture (Cytodex-1, Cytodex-3).

**Table 4. Oligosaccharides produced by COS-7 cells**

| | | |
|---|---|---|
| (X1) | C₁₂-Glc-Gal-NeuAc | (GM3 type) |
| (X2) | C12-Glc-Gal-GalAc ; NeuAc | (GM2 type) |
| (X3) | C12-Glc-Gal-GalAc-Gal ; NeuAc | (GM1 type) |
| (X4) | C12-Glc-Gal-GalNAc-Gal-Hex ; NeuAc | (Hex-GM1 type) |
| | C12-Glc-Gal-GalNAc-Gal-HexNAc ; NeuAc | (HexNAc-GM1 type) |
| (X5) | C12-Glc-Gal-GalNAc-Gal-NeuAc ; NeuAc | (GD1a type) |

### Example 5: Preparation of a lactoside type primer

A saccharide primer 1-O-dodecyl-4-O-β-D-galactopyranosyl-β-D-glucopyranoside was synthesized according to the following reaction scheme.

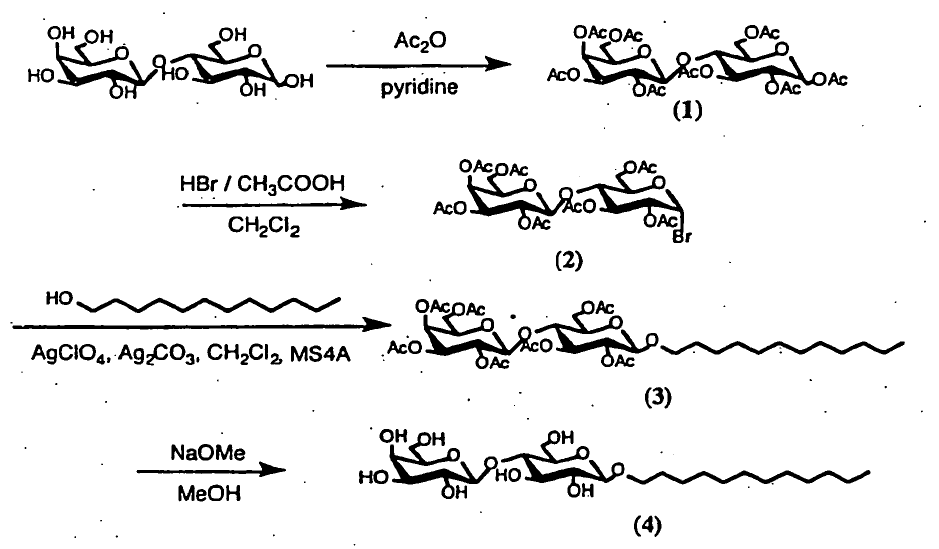

### (a) Synthesis of lactose octaacetate (1)

10g (29 mmol, Sigma) of lactose, 160 ml (1.8 mol, nacalai tesque) of acetic anhydride, and 160 ml (nacalai tesque) of pyridine were placed into a Erlenmeyer flask, and stirred overnight at room temperature. After confirming the completion of reaction by TLC, the reaction mixture was poured into distilled water on ice (a brown sticky substance precipitated), and stirred overnight. The precipitate formed was washed in distilled water, and the neutralization of the supernatant was confirmed by a pH test paper, and then the precipitate was vacuum dried to obtain a white powder.
Yield, 12.4 g (63%).

### (b) Synthesis of 1-bromo-4-O-(2,3,4,6-tetra-O-acetyl-β-D-galactosyl)-2,3,6-tri-O-acetyl-β-D-glucopyranoside (2)

12 g (18 mmol) of compound (1), 20 ml of 25% hydrogen bromide/acetic acid solution (Wako) and dehydrated dichloromethane were stirred for 1 hour under cooling on ice. After confirming the completion of reaction by TLC, chloroform was added, and neutralized with a saturated sodium bicarbonate solution. After the chloroform layer was washed with water and dried with sodium sulfate, the solution was evaporated and vacuum dried (a white solid formed on the wall of the flask). The solid was dissolved in ethyl acetate, to which hexane was added dropwise and the white solid formed was recovered, and vacuum dried to obtain a white powder. Yield, 7.6 g (61%).
¹H-NMR (CDCl₃, TMS): δ 2.2-2.2 (m, 21H, O-acetyl group), 3.9-4.2 (m, 6H, H-4, H-5, H-6, H'-5, H'-6, H'-6'), 4.48 (m, 1H, H7-6'), 4.52 (d, 1H, H'-1, J12=7.8 Hz, β-anomer), 4.8 (dd, 1H, H-2), 5.0 (dd, 1H, H'-3), 5.1 (dd, 1H, H'-2), 5.4 (d, 1H, H'-4), 5.6 (t, 1H, H-3), 6.5 (d, 1H, H-1, J₁₂=3.2 Hz, α-anomer)

### (c) Synthesis of 1-O-n-dodecyl-4-O-(2,3,4,6-tetra-O-acetyl-β-D-galactosyl)-2,3,6-tri-O-acetyl-β-D-glucopyranoside (3)

3.0 g of activated molecular sieve 4A (nacalai tesque), 5.0 g of compound (2) (6.8 mmol), and dehydrated dichloromethane were placed into a pear-shaped flask, and stirred in Ar gas for 2 hours. At the same time, 5.0 g of molecular sieve 4A, 1.9 g of n-decanol (10 mmol, nacalai tesque), 1.4 g (10.0 mmol, nacalai tesque) of silver perchlorate, 1.9 g (10.0 mmol, Wako) of silver carbonate, and 50 ml of dehydrated dichloromethane were placed in a light-tight pear-shaped flask, and stirred in Ar gas for 2 hours. Then, the content of the flask having compound (2) was transferred to a light-tight flask on an ice bath, and stirred as it was in Ar gas for 15 hours. Molecular sieve and silver salts were removed by filtering through Celite, and the filtrate was evaporated. Purification was carried out using an open column chromatography (Silica Gel 60, Merck, φ 5 x 20 cm, eluent: n-hexane : ethyl acetate = 55:45 → 50:50). Fractions containing the product of interest were collected and concentrated to yield a yellow sticky substance.
Yield 1.8 g (32%)
Analysis MALDI-TOF MS calcd. [M+Na]⁺= 827.37, found [M+Na]⁺= 827.80
The result of ¹H-NMR is shown on the next page.

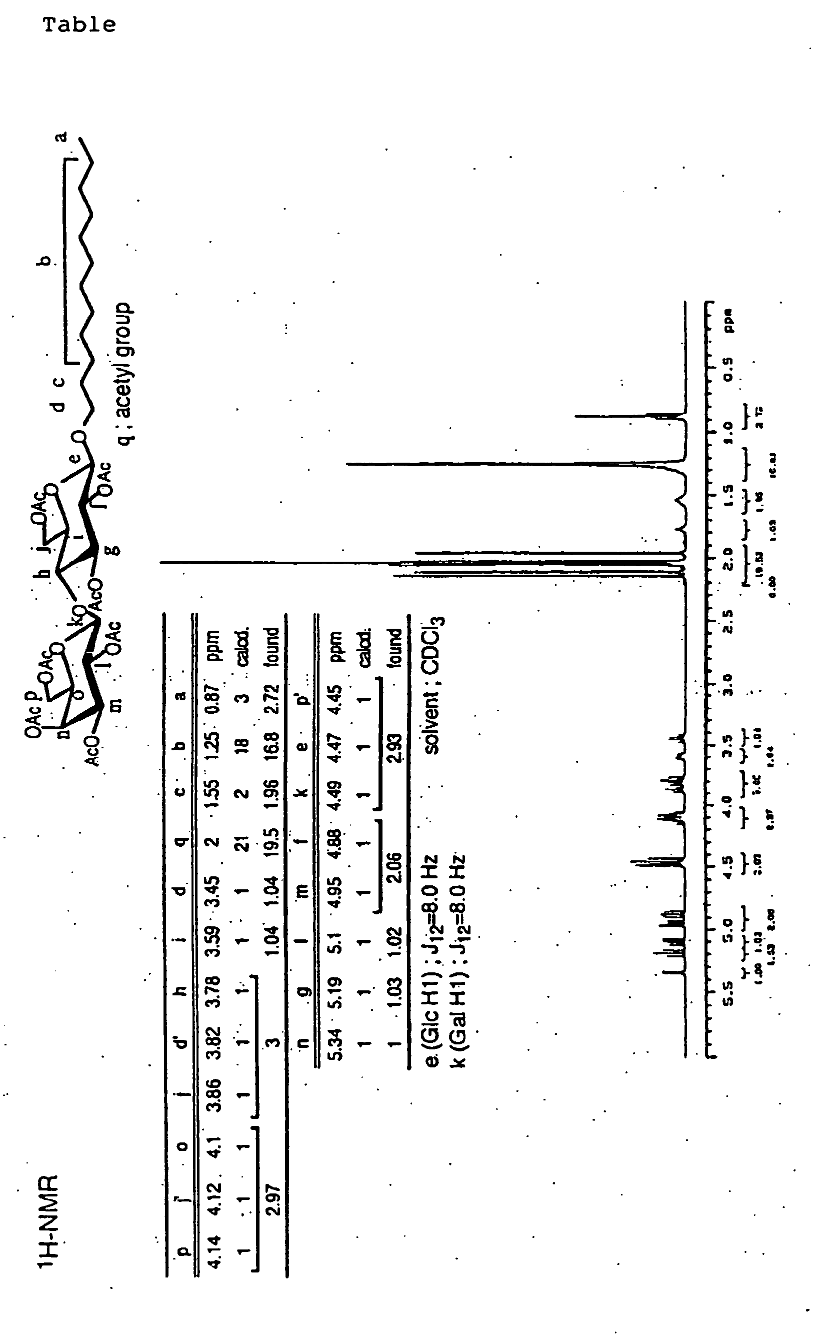

### (d) Synthesis of 1-O-n-dodecyl-4-O-(β-D-galactosyl)-β-D-glucopyranoside (4)

1.5 g of compound (3) was dissolved in 50 ml of methanol, and 100 mg of sodium methoxide was added thereto and stirred at room temperature for 3 hours. During the stirring, methanol was added to render the system homogeneous. After the reaction is over, Amberlite 1R-120R (Organo) was used for ion exchange. After confirming the neutralization, it was filtered to remove the resin. The filtrate was evaporated and vacuum dried to yield a white powder.
Yield, 650 mg (68%)
MALDI-TOF MS calcd. [M+Na]'= 533.28, found [M+Na]⁺= 533.52
¹H-NMR (DMSO-d₆) : δ 0.85 (t, 3H, CH₂CH₃), 1.25 (m, 18H, (CH₂)₁₀CH₃), 1.50 (quint, 2H, OCH₂CH₂), 4.115 (d, 1H, H-1, J₁₂= 8.0 Hz, β-anomer) 4.20(d,1H, H'-1, J₁₂= 8.8 Hz, β-anomer)

### Industrial Applicability

According to the present invention, the production of oligosaccharides by giving saccharide primers to human tissue-derived normal cells cultured using a high-density culture method has become possible.

By allowing cells to produce oligosaccharides, every functional oligosaccharide present in living organisms can be obtained. Oligosaccharides are involved in development, differentiation, propagation, cell death, or toxins, viruses, bacterial infection, and further tumor markers and metastasis. Recently, receptors of amyloid proteins that are causative agents for Alzheimer's disease are being considered to be sugar chains. It is also possible to use saccharide library as material, and to discover oligosaccharides having the highest activity as inhibitors thereof. Alternatively, glyco chips can also be generated by constructing a saccharide library and immobilizing it onto a microplate. The preparation of such glyco chips conceivably could be used in applications of biotechnology-related research and development including not only the analysis of molecular functions such as receptor analysis in the field of biochemistry, molecular biology, cell engineering, virology, and the like, but also as test reagents for detecting tumor markers and toxins in the clinical field.

## Claims

1. A method for producing oligosaccharides comprising giving a saccharide primer to human tissue-derived non-embryonic normal cells, and culturing the cells using a high-density culture method.

2. The method of claim 1, wherein the human tissue-derived normal cells are diploid fibroblasts or vascular endothelial cells.

3. The method of claim 1 or claim 2, wherein the high-density culture method is a microcarrier culture method, a culture tank using a cell immobilisation disk, a culture system using a hollow fibre module, or a suspension culture of suspended cells.

4. The method of claim 3, wherein the high-density culture method is a microcarrier culture method.

5. The method of any preceding claim, wherein the saccharide primer is Gal-Glc-C12.

6. The method of claim 5, wherein the human cells are diploid fibroblasts and the method is for producing the following oligosaccharides:
| | |
|---|---|
| Gal-Gal-Glc-C12 | (Gb3 type) |
| GalNAc-Gal-Gal-Glc-C12 | (Gb4 type) |
| NeuNAc-GalNAc-Gal-Gal-Glc-C12 | (NeuNAc-Gb4 type) |
| NeuNAc-Gal-Glc-C12 | (GM3 type) |
| NeuNAc-NeuNAc-Gal-Glc-C12 | (GD3 type) |

7. The method of claim 5, wherein the human cells are vascular endothelial cells and the method is for producing the following oligosaccharides:
| | |
|---|---|
| Gal-Gal-Glc-C12 | (Gb3 type) |
| GalNAc-Gal-Gal-Glc-C12 | (Gb4 type) |
| NeuNAc-GalNAc-Gal-Gal-Glc-C12 | (NeuNAc-Gb4 type) |
| NeuNAc-Gal-Glc-C12 | (GM3 type) |

## Patentansprüche

1. Verfahren zur Herstellung von Oligosacchariden, umfassend das Hinzugeben eines Saccharidprimers zu von menschlichem Gewebe stammenden, nichtembryonischen Normalzellen und Züchten der Zellen unter Verwendung eines Kulturverfahrens mit hoher Zellkonzentration.

2. Verfahren nach Anspruch 1, worin die von menschlichem Gewebe stammenden Normalzellen diploide Fibroblasten oder Gefäßendothelzellen sind.

3. Verfahren nach Anspruch 1 oder Anspruch 2, worin das Kulturverfahren mit hoher Zellkonzentration ein Mikroträger-Kulturverfahren, ein Kulturtank unter Verwendung einer Zellenimmobilisierungsplatte, ein Kultursystem unter Verwendung eines Hohlfasermoduls oder eine Suspensionskultur suspendierter Zellen ist.

4. Verfahren nach Anspruch 3, worin das Kulturverfahren mit hoher Zellkonzentration ein Mikroträger-Kulturverfahren ist.

5. Verfahren nach einem der vorangegangenen Ansprüche, worin der Saccharidprimer Gal-Glc-C12 ist.

6. Verfahren nach Anspruch 5, worin die menschlichen Zellen diploide Fibroblasten sind und das Verfahren dem Produzieren der folgenden Oligosaccharide dient:
| | |
|---|---|
| Gal-Gal-Glc-C12 | (Gb3-Typ) |
| GaINAc-Gal-Gal-Glc-C12 | (Gb4-Typ) |
| NeuNAc- GaINAc-Gal-Gal-Glc-C12 | (NeuNAc-Gb4-Typ) |
| NeuNAc-Gal-Glc-C12 | (GM3-Typ) |
| NeuNAc-NeuNAc-Gal-Glc-C12 | (GD3-Typ). |

7. Verfahren nach Anspruch 5, worin die menschlichen Zellen Gefäßendothelzellen sind und das Verfahren dem Produzieren der folgenden Oligosaccharide dient:
| | |
|---|---|
| Gal-Gal-Glc-C12 | (Gb3-Typ) |
| GalNAc-Gal-Gal-Glc-C12 | (Gb4-Typ) |
| NeuNAc- GaINAc-Gal-Gal-Glc-C12 | (NeuNAc-Gb4-Typ) |
| NeuNAc-Gal-Glc-C12 | (GM3-Typ). |

## Revendications

1. Méthode de production d'oligosaccharides comprenant la fourniture d'une amorce de saccharides à des cellules normales humaines non-embryonnaires dérivées du tissu, et la culture des cellules en utilisant une méthode de culture haute densité.

2. Méthode selon la revendication 1, dans laquelle les cellules normales humaines dérivées du tissu sont des fibroblastes diploïdes ou des cellules endothéliales vasculaires.

3. Méthode selon la revendication 1 ou la revendication 2, dans laquelle la méthode de culture haute densité est une méthode de culture microsupport, une cuve de culture utilisant un disque d'immobilisation des cellules, un système de culture utilisant un module de fibres creuses ou une culture de suspension de cellules suspendues.

4. Méthode selon la revendication 3, dans laquelle la méthode de culture haute densité est une méthode de culture microsupport.

5. Méthode selon l'une quelconque des revendications précédentes, dans laquelle l'amorce de saccharides est Gal-Glc-C12.

6. Méthode selon la revendication 5, dans laquelle les cellules humaines sont des fibroblastes diploïdes, et la méthode est pour la production des oligosaccharides suivants:
| | |
|---|---|
| Gal-Gal-Glc-C12 | (type Gb3) |
| GalNAc-Gal-Gal-Glc-C12 | (type Gb4) |
| NeuNAc-GalNAc-Gal-Gal-Glc-C12 | (type NeuNAc-Gb4) |
| NeuNAc-Gal-Glc-C12 | (type GM3) |
| NeuNAc-NeuNAc-Gal-Glc-C12 | (type GD3) |

7. Méthode selon la revendication 5, dans laquelle les cellules humaines sont des cellules endothéliales vasculaires, et la méthode est pour la production des oligosaccharides suivants:
| | |
|---|---|
| Gal-Gal-Glc-C12 | (type Gb3) |
| GalNAc-Gal-Gal-Glc-C12 | (type Gb4) |
| NeuNAc-GalNAc-Gal-Gal-Glc-C12 | (type NeuNAc-Gb4) |
| NeuNAc-Gal-Glc-C12 | (type GM3) |
